(19) **Europäisches Patentamt**

**European Patent Office**

**Office européen des brevets**

(11) **EP 0 876 135 B1**

(12) **EUROPÄISCHE PATENTSCHRIFT**

(45) Veröffentlichungstag und Bekanntmachung des Hinweises auf die Patenterteilung:
**09.04.2003 Patentblatt 2003/15**

(21) Anmeldenummer: **97910385.0**

(22) Anmeldetag: **29.09.1997**

(51) Int Cl.⁷: **A61K 7/13**

(86) Internationale Anmeldenummer:
**PCT/EP97/05340**

(87) Internationale Veröffentlichungsnummer:
**WO 98/017233 (30.04.1998 Gazette 1998/17)**

(54) **FÄRBEMITTEL ZUR FÄRBUNG VON KERATINISCHEN FASERN**

DYEING AGENT FOR DYEING KERATIN FIBRES

MATIERE COLORANTE POUR LA COLORATION DE FIBRES KERATINIQUES

(84) Benannte Vertragsstaaten:
**DE ES FR GB IT**

(30) Priorität: **18.10.1996 DE 19643058**

(43) Veröffentlichungstag der Anmeldung:
**11.11.1998 Patentblatt 1998/46**

(73) Patentinhaber: **Wella Aktiengesellschaft**
**64274 Darmstadt (DE)**

(72) Erfinder: **BRAUN, Hans-Jürgen**
**CH-3182 Ueberstorf (CH)**

(56) Entgegenhaltungen:
**EP-A- 0 706 787          DE-A- 3 834 142**
**DE-A- 3 942 294**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft eine Zusammensetzung zur Färbung von keratinischen Fasern, insbesonders von menschlichen Haaren. Diese Zusammensetzung enthält eine Kombination aus einem p-Aminophenol, einem m-Aminophenol, einem p-Phenylendiamin und einem 2-Amino-6-chlor-nitrophenol. Die Färbungen der keratinischen Fasern werden ausgeführt, indem die oben erwähnte Zusammensetzung mit einem Oxidationsmittel vermischt wird, auf die keratinischen Fasern aufgetragen wird und nach einer bestimmten Einwirkungszeit mit Wasser und einem Shampoo wieder ausgewaschen wird.

Für eine bestimmte Gruppe von Anwendungen besteht eine Nachfrage nach Zusammensetzungen, die Haare in kupferfarbigen, kastanienbraunen bis roten Nuancen zu färben vermögen. Diese Nachfrage wird heute befriedigt, indem auf dem Markt Haarfärbemittel mit einem Gehalt an bestimmten Oxidatiorisfarbstoffvorstufen angeboten werden. Beispielsweise wird in der DE-OS 36 10 396 zur Erzeugung von neutralen roten Farbtönen die Verwendung einer Kombination aus 5-Amino-2-methylphenol, 4-Amino-3-methylphenol und 1,4-Diaminobenzol und/oder 2,5-Diaminotoluol angegeben. In der EP-OS 0 634 162 werden Beispiele zur Kombination von 5-Amino-2-methylphenol und dessen N-substituierten Derivaten, 4-Amino-2-methylphenol, 4-Amino-3-methylphenol oder 4-Amino-2-hydroxymethylphenol und bestimmten m-Phenylendiaminderivaten angegeben.

In der EP-OS 0 634 164 sind dagegen Kombinationen aus p-Aminophenolderivaten, 5-Amino-2-methylphenol und p-Phenylendiaminderivaten beschrieben. In der EP-OS 0 706 787 werden unter anderem Färbemittel beschrieben, die neben 2-Chlor-6-ethylamino-4-nitrophenol eine Kombination aus 1,4-Diaminobenzol und/oder 2,5-Diaminototuoi sowie 2-(2',5'-Diaminophenyl)ethanol und 5-Amino-2-methyl-phenol enthalten.

Ein Problem besteht in der Praxis bezüglich des unterschiedlichen Aufziehverhaltens der Farbstoffe in Abhängigkeit von der Haarbeschaffenheit, wodurch eine ungleichmäßige Anfärbung der Haare erfolgt. Normalerweise neigen die verwendeten Farbstoffe dazu, auf geschädigte Haarpartien stärker aufzuziehen als auf ungeschädigte Haarpartien. Aus diesem Grunde werden in der Regel die durch übliche Alterungsprozesse und Umwelteinflüsse (zum Beispiel Sonnenlicht, Haarwäsche, Färbe- und Dauerwellbehandlungen) stärker geschädigten Haarspitzen intensiver gefärbt als die weniger geschädigten Haarlängen und der Haaransatz, wodurch ein unnatürliches, ungleichmäßiges und völlig unbefriedigendes Färbeergebnis erhalten wird. Dieses Verhalten macht sich besonders bei hellen Nuancen sehr störend bemerkbar.

Es bestand daher die Aufgabe, ein Oxidationshaarfärbemittel zur Verfügung zu stellen, das eine gleichmäßige und farbsatte Färbung der Haare von den Haarspitzen bis zum Haaransatz im Naturbereich bis zu modischen Färbungen mit orangen bis rötlichen Reflexen ermöglicht.

Die in der EP-OS 0 634 162, der EP-OS 0 634 164 und der EP-OS 0 706 787 beschriebenen Haarfärbemittel erfüllen die Anforderungen bezüglich eines gleichmäßigen Farbresultates vom Haaransatz bis zu den Haarspitzen jedoch nicht.

Ueberraschend wurde nunmehr gefunden, daß mit einer eine Kombination aus

- mindestens einem p-Aminophenolderivat, welches ausgewählt ist aus 4-Amino-2-methylphenol, 4-Amino-3-methylphenol, 4-Amino-2-hydroxymethyl-phenol, 4-Amino-2-aminomethyl-phenol und 4-Amino-2-((2'-hydroxyethyl)aminomethyl)-phenol,
- mindestens einem p-Phenylendiaminderivat, welches ausgewählt ist aus 2-(2',5'-Diaminophenyl)ethanol, 2-Hydroxymethyl-p-phenylendiamin, 2-Propyl-p-phenylendiamin, 2-Isopropyl-p-phenylendiamin und N-N-Bis(2'-hydroxyethyl)-p-phenylendiamin,
- mindenstens einem m-Aminophenolderivat, welches ausgewählt ist aus 5-Amino-2-methylphenol, 5-((2'-Hydroxyethyl)amino)-2-methylphenol und 5-((3'-Hydroxypropyl)amino)-2-methylphenol und
- mindestens einem 2-Amino-6-chlor-nitrophenolderivat, welches ausgewählt ist aus 2-Amino-6-chlor-4-nitrophenol, 6-Chlor-2-ethylamino-4-nitrophenol und 6-Chlor-2-((2'-hydroxyethyl)amino)-4-nitrophenol, enthaltenden Farbträgermasse Haarfärbemittel hergestellt werden können, mit denen die beschriebenen Probleme bezüglich des Farbausgleichs zwischen Haaransatz und Haarspitze erfolgreich gelöst werden können.

Die beschriebenen Farbstoffkombinationen ermöglichen eine vom Haaransatz bis zur Haarspitze gleichmäßige Färbung im Naturbereich bis zu leicht modischen Färbungen mit rötlichen Reflexen, ganz besonders auch bei helleren Nuancen. Die vorzüglichen Eigenschaften der neuen Farbstoffkombination zeigen sich besonders auf durch Licht und Wetter geschädigtem oder auf dauergewellten Haaren.

Zur Abrundung des Farbergebnisses sowie zur Erzeugung von speziellen Farbeffekten können dem Haarfärbemittel weitere Oxidationsfarbvorstufen wie Derivate des m- oder p-Phenylendiamins sowie direktziehende Farbstoffe, wie zum Beispiel Azofarbstoffe, Anthrachinone oder Nitrobenzolderivate zugesetzt werden.

Die vorstehend beschriebene erfindungsgemäße Kombination von oxidativen Haarfarbvorstufen und gegebenenfalls direktziehenden Farbstoffen werden zur Färbung ein einer geeigneten Farbträgermasse appliziert.

Der Gegenstand der vorliegenden Anmeldung betrifft daher weiterhin ein Mittel zur oxidativen Färbung von Haaren, welches durch Vermischen der erfindungsgemäßen Farbträgermasse mit einem Oxidationsmittel unmittelbar vor der Anwendung hergestellt wird.

Die erfindungsgemäße Farbträgermasse enthält die vorstehend beschriebenen erfindungsgemäßen Kombinationen als solche oder in Form von physiologisch verträglichen Salzen, beispielsweise als Hydrochloride, Sulfate oder Tartrate, oder im Fall von Phenolen als Alkaliphenolate.

Die Gesamtkonzentration an Farbvorstufen beträgt 0,1 und 10 Gewichtsprozent, vorzugsweise 0,2 und 6 Gewichtsprozent.

Darüberhinaus können in der Farbträgermasse noch übliche kosmetische Zusätze, beispielsweise Antioxidantien wie Ascorbinsäure, Thioglykolsäure oder Natriumsulfit; Parfümöle; Komplexbildner; Netzmittel; Emulgatoren; Verdicker; Pflegestoffe und andere vorhanden sein.

Die Zubereitungsform für die Farbträgermasse sowie für das gebrauchsfertige Oxidationshaarfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Das gebrauchsfertige erfindungsgemäße Haarfärbemittel wird durch Mischen der Farbträgermasse mit einem flüssigen Oxidationsmittel unmittelbar vor der Anwendung hergestellt.

Die Farbträgermasse und das Oxidatiönsmittel werden hierbei im Gewichtsverhältnis von 5:1 bis 1:3 miteinander vermischt, wobei ein Gewichtsverhältnis von 1:1 bis 1:2 besonders bevorzugt ist.

Des pH-Wert des gebrauchsfertigen erfindungsgemäßen Haarfärbemittel stellt sich bei der Mischung der vorzugsweise alkalisch eingestellten Farbträgermasse mit dem meist sauer eingestellten Oxidationsmittel auf einen pH-Wert ein, der durch die Alkalimengen in der Farbträgermasse und die Säuremengen im Oxidationsmittel sowie durch das Mischungsverhältnis beinflußt wird. Der pH-Wert des fertigen Haarfärbemittels kann bei 3 bis 11, vorzugsweise 5 bis 9 liegen.

Für die Einstellung des jeweiligen pH-Wertes der Farbträgermasse und des Oxidationsmittels können je nach dem gewünschten pH-Wert organische und anorganische Säuren, wie zum Beispiel Phosphorsäure, Ascorbinsäure und Milchsäure, oder Alkalien wie zum Beispiel Monoethanolamin, Triethanolamin, 2-Amino-2-methyl-1-propanol, Ammoniak, Natronlauge, Kalilauge oder Tris(hydroxymethyl)-amino-methan, verwendet werden.

Für die Anwendung zur oxidativen Färbung von Haaren vermischt man die vorstehend beschriebene Farbträgermasse unmittelbar vor dem Gebrauch mit einem Oxidationsmittel und trägt eine für die Haarfärbebehandlung ausreichende Menge, je nach Haarfülle im allgemeinen etwa 60 bis 200 Gramm des erhaltenen gebrauchsfertigen Oxidationshaarfärbemittels auf das Haar auf.

Als Oxidationsmittel kommen hauptsächlich Wasserstoffperoxid oder dessen Additionsverbindungen an Harnstoff, Melamin oder Natriumbromat in Form einer 1 bis 12prozentigen, vorzugsweise 6prozentigen, wäßrigen Lösung in Betracht, wobei Wasserstoffperoxid besonders bevorzugt wird.

Man lässt das erfindungsgemäße Haarfärbemittel bei 15 bis 50 Grad Celsius etwa 10 bis 45 Minuten lang, vorzugsweise 30 Minuten lang, auf das Haar einwirken, spült sodann das Haar mit Wasser und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die nachfolgenden Beispiele sollen den Gegenstand näher erläutern, ohne ihn auf diese Beispiele zu beschränken.

**Beispiele**

**Beispiele 1 bis 6:** Haarfärbelösungen bei einem basischen pH-Wert

Es wird folgende Farbelösung hergestellt:

| | |
|---|---|
| 10,0 g | Isopropanol |
| 10,0 g | Laurylalkohol-diglykolether-sulfat-Natriumsalz, 28prozentige wäßrige Lösung |
| 10,0 g | Ammoniak, 25prozentige wäßrige Lösung |
| 0,3 g | Ascorbinsäure |
| X g | Farbvorstufen gemäß Tabelle 1 |
| ad 100 g | Wasser vollentsalzt |

Zur Anwendung werden 10 g Haarfärbelösung mit 10 g Wasserstoffperoxid-Lösung (6prozentige Lösung in Wasser) gemischt. Das erhaltene Oxidationshaarfärbemittel wird auf Haarsträhnchen aufgetragen. Nach einer Einwirkungszeit von 30 Minuten bei 40 °C werden die Haare mit Wasser gespült, schamponiert und getrocknet.

Tabelle 1

| Farbvorstufen / Beispiel | 1 | 2 | 3 | 4 | 5 | 6 |
|---|---|---|---|---|---|---|
| 4-Amino-2-methylphenol | - | 0,5 g | - | - | 0,1 g | - |
| 4-Amino-3-methylphenol | 1,0 g | - | 1,0 g | 2,0 g | 0,1 g | 0,1 g |
| 4-Amino-2-hydroxymethyl-phenol | - | 0,5 g | - | - | - | - |
| 4-Amino-2-aminomethyl-phenol-dihydrochlorid | - | - | 0,1 g | - | - | - |
| 2-(2',5'-Diaminophenyl)-ethanolsulfat | 0,1 g | 0,1 g | - | - | 0,1 g | 0,1 g |
| 2-Hydroxymethyl-p-phenylendiamin-sulfat | - | 0,1 g | - | - | - | - |
| N,N-Bis(2-hydroxyethyl)-p-phenylen-diamin | 0,15 g | - | 0,2 g | 0,1 g | - | - |
| 5-Amino-2-methylphenol | 1,5 g | 1,4 g | 1,2 g | 1,5 g | 0,5 g | 0,15 g |
| 5-((2'-Hydroxyethyl)amino)-2-methyl-phenol | - | 0,1 g | - | 0,4 g | - | - |
| 5-((3'-Hydroxypropyl)amino)-2-methyl-phenol | - | 0,1 g | - | - | - | - |
| 2-Amino-6-chlor-4-nitrophenol | - | 0,05 g | 0,05 g | 0,1 g | 0,2 g | - |
| 2-Chlor-6-ethylamino-4-nitrophenol | 0,2g | - | 0,2 g | - | - | 0,1 g |
| 6-Chlor-2-((2'-hydroxyethyl)-amino)-4-nitrophenol | - | 0,1 g | - | - | - | - |
| **Haarfärbung auf Tierhaar** | ziegel-rot | ziegel-rot | rot-braun | ziegel-rot | orange rot | lachs-rot |

**Beispiele 7 bis 12:** Haarfärbelösungen bei einem basischen pH-Wert

Es wird folgende Farbelösung hergestellt:

| | |
|---|---|
| 10,0 g | Isopropanol |
| 10,0 g | Laurylalkohol-diglykolether-sulfat-Natriumsalz, 28prozentige wäßrige Lösung |
| 10,0 g | Ammoniak, 25prozentige wäßrige Lösung |
| 0,3 g | Ascorbinsäure |
| 0,1 g | Natriumsulfit wasserfrei |

(fortgesetzt)

| X g | Farbvorstufen gemäß Tabelle 1 |
|---|---|
| ad 100 g | Wasser vollentsalzt |

Zur Anwendung werden 10 g Haarfärbelösung mit 10 g Wasserstoffperoxid-Lösung (6prozentige Lösung in Wasser) gemischt Das erhaltene Oxidationshaarfärbemittel wird auf Haarsträhnchen aufgetragen. Nach einer Einwirkungszeit von 30 Minuten bei 40 °C werden die Haare mit Wasser gespült, schamponiert und getrocknet.

Tabelle 2

| Farbvorstufen / Beispiel | 7 | 8 | 9 | 10 | 11 | 12 |
|---|---|---|---|---|---|---|
| 4-Amino-3-methylphenol | - | 0,5 g | - | 0,5 g | 2,0 g | 1,5 g |
| 4-Amino-2-hydroxymethyl-phenol | - | - | 0,5 g | - | - | - |
| 4-Amino-2-aminomethyl-phenol-dihydrochlorid | 1,5 g | 0,2 g | - | - | - | - |
| 2-(2,5-Diaminophenyl)-ethanolsulfat | 0,3 g | 0,1 g | - | 0,15 g | 0,1 g | 2,0 g |
| N,N-Bis(2-hydroxyethyl)-p-phenylendiamin | - | 0,1 g | 0,4 g | 0,3 g | - | 0,2 g |
| 5-Amino-2-methylphenol | 0,4 g | 0,7 g | 0,6 g | 0,8 g | 2,1 g | 3,0 g |
| 2-Amino-6-chlor-4-nitrophenol | - | 0,1 g | - | - | - | - |
| 6-Chlor-2-ethylamino-4-nitrophenol | 0,2 g | 0,1 g | 0,2 g | 0,3 g | 0,01 g | 0,16 g |
| **Haarfarbe auf Tierhaar** | orange-braun | rot-braun | rot-braun | glut-rot | helles Ziegelrot | wein-rot |

Die vorstehenden Färbungen fallen unabhängig von der Haarschädigung sehr gleichmäßig aus.

**Beispiel 13:** Haarfärbemittel in Cremeform

| 0,34 g | 2,5-Diaminophenylethylalkohol-sulfat |
|---|---|
| 1,31 g | 4-Amino-2-aminomethyl-phenol-hydrochlorid |
| 0,30 g | m-Aminophenol |
| 1,5 g | 5-Amino-2-methyl-phenol |
| 0,15 g | 2-Amino-6-chlor-4-nitrophenol |
| 15,00 g | Cetylalkohol |
| 3,50 g | Natrium-Laurylalkohol-diglykolethersulfat, 28prozentige wäßrige Lösung |
| 3,00 g | Ammoniak, 25prozentige wäßrige Lösung |
| 0,30 g | Natriumsulfit, wasserfrei |
| 74,60 g | Wasser |
| 100,00 g | |

10 g dieses Haarfärbemittels werden kurz vor Gebrauch mit 10 ml Wasserstoffperoxidlösung (6prozentig) vermischt. Anschließend trägt man das Gemisch auf blonde Naturhaare auf und läßt es 30 Minuten lang bei 40 Grad Celsius einwirken. Danach wird das Haar mit Wasser gespült und getrocknet. Das Haar hat eine gleichmäßig kastanienbraune Färbung erhalten.

**Beispiel 14:** Haarfärbemittel in Gelform

| | |
|---|---|
| 1,5 g | 5-Amino-2-methyl-phenol |
| 1,0 g | 4-Amino-2-aminomethyl-phenol-dihydrochlorid |
| 0,4 g | N,N-Bis(2'-hydroxyethyl)-p-phenylendiamin |
| 0,5 g | 6-Chlor-2-ethylamino-4-nitrophenol |
| 0,4 g | Natriumhydroxid, fest |
| 0,6 g | Ascorbinsäure |
| 7,0 g | Isopropanol |
| 15,0 g | Ölsäure |
| 10,0 g | Ammoniak, 25prozentige wäßrige Lösung |
| 63,6 g | Wasser |
| 100,0 g | |

Man vermischt kurz vor dem Gebrauch 50 g dieses Haarfärbemittels mit 50 ml Wasserstoffperoxidlösung (6prozentig) und läßt das Gemisch 30 Minuten lang auf blonde menschliche Haare einwirken. Danach wird mit Wasser gespült und sodann getrocknet. Man erhält ein vom Haaransatz bis in die Haarspitzen gleichmäßiges dunkles Braunorange.

**Beispiel 15:** Haarfärbelösung mit saurem pH-Wert

| | |
|---|---|
| 0,30 g | 2-(2',5'-Diaminophenyl)ethanol-sulfat |
| 0,30 g | 4-Amino-2-aminomethylphenol-dihydrochlorid |
| 0,30 g | 5-Amino-2-methylphenol |
| 0,30 g | Ascorbinsäure |
| 0,28 g | Natriumlaurylethersulfat |
| 0,22 g | Ammoniak, 25prozentige wäßrige Lösung |
| 98,30 g | Wasser |
| 100,00 g | |

Der pH-Wert der Farbträgermasse wird mit verdünnter Phosphorsäure oder einer verdünnten Ammoniaklösung auf einen pH-Wert von 6,8 eingestellt.

Unmittelbar vor der Anwendung werden 20 Gramm der Haarfärbelösung mit 20 Gramm einer 6prozentigen, wäßrigen Wasserstoffperoxidlösung (pH = 6,8) vermischt und das erhaltene Oxidationshaarfärbemittel (pH = 6,8) auf gebleichte Haare aufgetragen. Nach einer Einwirkungszeit von 30 Minuten bei Raumtemperatur werden die Haare mit Wasser gespült und getrocknet.

Das so behandelte Haar ist in einem gleichmäßig hellen Aubergineton gefärbt.

**Patentansprüche**

1.   Haarfarbträgermasse **dadurch gekennzeichnet, daß** sie eine Kombination von

-   mindestens einem p-Aminophenolderivat, welches ausgewählt ist aus 4-Amino-2-methylphenol, 4-Amino-3-methylphenol, 4-Amino-2-hydroxymethyl-phenol, 4-Amino-2-aminomethyl-phenol und 4-Amino-2-((2'-hydroxyethyl)aminomethyl)-phenol,
-   mindestens einem p-Phenylendiaminderivat, welches ausgewählt ist aus 2-(2',5'-Diaminophenyl)ethanol, 2-Hydroxymethyl-p-phenylendiamin, 2-Propyl-p-phenylendiamin, 2-Isopropyl-p-phenylendiamin und N-N-Bis (2'-hydroxyethyl)-p-phenylendiamin,
-   mindenstens einem m-Aminophenolderivat, welches ausgewählt ist aus 5-Amino-2-methylphenol, 5-((2'-Hydroxyethyl)amino)-2-methylphenol und 5-((3'-Hydroxypropyl)amino)-2-methylphenol, und

- mindestens einem 2-Amino-6-chlor-nitrophenolderivat, welches ausgewählt ist aus 2-Amino-6-chlor-4-nitrophenol, 6-Chlor-2-ethylamino-4-nitrophenol und 6-Chlor-2-((2'-hydroxyethyl)amino)-4-nitrophenol, enthält.

**2.** Haarfarbträgermasse nach Anspruch 1, **dadurch gekennzeichnet, daß** die Farbstoffe in einer Gesamtmenge von 0,1 bis 10 Gewichtsprozent enthalten sind.

**3.** Haarfarbträgermasse nach Anspruch 1 oder 2, **dadurch gekennzeichnet, daß** sie übliche kosmetische Zusätze enthält.

**4.** Haarfärbemittel, **dadurch gekennzeichnet, daß** es unmittelbar vor der Anwendung durch Vermischen einer Haarfarbträgermasse gemäß einem der Ansprüche 1 bis 3 mit einem Oxidationsmittel hergestellt wird.

**5.** Haarfärbemittel nach Anspruch 4, **dadurch gekennzeichnet, daß** die Haarfarbträgermasse mit dem Oxidationsmittel in einem Verhältnis von 5:1 bis 1:3 vermischt wird.

**6.** Haarfärbemittel nach Anspruch 4 oder 5, **dadurch gekennzeichnet, daß** es einen pH-Wert von 3 bis 11 aufweist.

**7.** Verfahren zum Färben von Haaren, **dadurch gekennzeichnet, daß** ein Haarfärbemittel nach einem der Ansprüche 4 bis 6 auf das Haar aufgetragen wird, bei einer Temperatur von 15 bis 50 °C 10 bis 45 Minuten lang einwirken gelassen wird, das Haar anschließend mit Wasser gespült wird, gegebenenfalls schamponiert wird, und sodann getrocknet wird.

**Claims**

**1.** Hair colour carrier mass **characterized in that** it comprises a combination of

- at least one p-aminophenol derivative which is chosen from 4-amino-2-methylphenol, 4-amino-3-methylphenol, 4-amino-2-hydroxymethylphenol, 4-amino-2-aminomethylphenol and 4-amino-2-((2'-hydroxyethyl)aminomethyl)phenol,
- at least one p-phenylenediamine derivative which is chosen from 2-(2',5'-diaminophenyl)-ethanol, 2-hydroxymethyl-p-phenylenediamine, 2-propyl-p-phenylenediamine, 2-isopropyl-p-phenylenediamine and N,N-bis(2'-hydroxyethyl)-p-phenylenediamine,
- at least one m-aminophenol derivative which is chosen from 5-amino-2-methylphenol, 5-((2'-hydroxyethyl)amino)-2-methylphenol and 5-((3'-hydroxypropyl)amino)-2-methylphenol, and
- at least one 2-amino-6-chloronitrophenol derivative which is chosen from 2-amino-6-chloro-4-nitrophenol, 6-chloro-2-ethylamino-4-nitrophenol and 6-chloro-2-((2'-hydroxyethyl)amino)-4-nitrophenol.

**2.** Hair colour carrier mass according to Claim 1, **characterized in that** the dyes are present in a total amount of from 0.1 to 10 percent by weight.

**3.** Hair colour carrier mass according to Claim 1 or 2, **characterized in that** it comprises customary cosmetic additives.

**4.** Hair colorant **characterized in that** it is prepared directly prior to application by mixing a hair colour carrier mass according to one of Claims 1 to 3 with an oxidizing agent.

**5.** Hair colorant according to Claim 4, **characterized in that** the hair colour carrier mass is mixed with the oxidizing agent in a ratio of from 5:1 to 1:3.

**6.** Hair colorant according to Claim 4 or 5, **characterized in that** it has a pH of from 3 to 11.

**7.** A method of colouring hair, **characterized in that** a hair colorant according to one of Claims 4 to 6 is applied to the hair, left to act at a temperature of from 15 to 50°C for 10 to 45 minutes, and the hair is then rinsed with water, optionally shampooed and then dried.

**Revendications**

1. Matière colorante pour cheveux, **caractérisée en ce qu'**elle contient une association d'

  - au moins un dérivé de p-aminophénol qui est choisi parmi le 4-amino-2-méthylphénol, le 4-amino-3-méthylphénol, le 4-amino-2-hydroxyméthylphénol, le 4-amino-2-aminométhylphénol et le 4-amino-2-((2'-hydroxyéthyl)aminométhyl)phénol,
  - au moins un dérivé de m-phénylènediamine qui est choisi parmi le 2-(2',5'-diaminophényl)éthanol, la 2-hydroxyméthyl-p-phénylènediamine, la 2-propyl-p-phénylènediamine, la 2-isopropyl-p-phénylènediamine et la N,N-bis(2'-hydroxyéthyl)-p-phénylènediamine,
  - au moins un dérivé de m-aminophénol qui est choisi parmi le 5-amino-2-méthylphénol, le 5-((2'-hydroxyéthyl)amino)-2-méthylphénol et le 5-((3'-hydroxypropyl)amino)-2-méthylphénol et
  - au moins un dérivé de 2-amino-6-chloronitrophénol qui est choisi parmi le 2-amino-6-chloro-4-nitrophénol, le 6-chloro-2-éthylamino-4-nitrophénol et le 6-chloro-2-((2'-hydroxyéthyl)amino)-4-nitrophénol.

2. Matière colorante pour cheveux selon la revendication 1, **caractérisée en ce que** les colorants sont contenus en une quantité totale de 0,1 à 10 % en poids.

3. Matière colorante pour cheveux selon la revendication 1 ou 2, **caractérisée en ce qu'**elle contient des additifs cosmétiques usuels.

4. Produit de teinture pour cheveux, **caractérisé en ce qu'**il est préparé immédiatement avant l'emploi, par mélange d'une matière colorante pour cheveux selon l'une quelconque des revendications 1 à 3, avec un oxydant.

5. Produit de teinture pour cheveux selon la revendication 4, **caractérisé en ce que** la matière colorante pour cheveux est mélangée avec l'oxydant en un rapport de 5:1 à 1:3.

6. Produit de teinture pour cheveux selon la revendication 4 ou 5, **caractérisé en ce qu'**il présente un pH de 3 à 11.

7. Procédé pour la teinture des cheveux, **caractérisé en ce qu'**on applique sur les cheveux un produit de teinture pour cheveux selon l'une quelconque des revendications 4 à 6, on laisse agir pendant 10 à 45 minutes à une température de 15 à 50°C, puis on rince les cheveux à l'eau, éventuellement on les shampooine et ensuite on les sèche.